# EUROPEAN PATENT SPECIFICATION

(11) **EP 4 611 860 B1**
(45) Date of publication and mention of the grant of the patent: **29.07.2026**
(21) Application number: 23828680.1
(22) Date of filing: 30.10.2023
(51) Int. Cl.: A61M 11/00, A61M 15/06, A24F 40/10

(54) **ATOMIZER**
ZERSTÄUBER
ATOMISEUR

(30) Priority: 04.11.2022 CN 202211380428
(43) Date of publication of application: 10.09.2025
(73) Proprietor: Shanghai QV Technologies Co., Ltd., Shanghai 201204 (CN)
(72) Inventor: DENG, Xiaogang, Shanghai 200120 (CN); LIU, Guiyou, Shanghai 200120 (CN); PENG, Xiaofeng, Shanghai 200120 (CN)
(74) Representative: Nederlandsch Octrooibureau
(86) International application number: PCT/CN2023/127580
(87) International publication number: WO 2024/093874

(56) References cited:
- CN-A- 115 104 780

## Description

### Technical Field

This invention generally relates to the field of atomization, in particular to an electronic atomizer.

### Prior Art

The atomization of this invention refers to atomizing liquid into aerosol which can be inhaled by people through the mouth and nose. Atomization functions widely in medical auxiliary equipment such as atomizing therapeutic apparatus, air quality improvement equipment for home and vehicles, electronic cigarettes and other products. The basic working principle of the atomizer is as follow: When negative pressure is applied to the nozzle, the air outside first enters into the atomizer throng the air inlet, and then flows out of the atomizer from the nozzle. While the air flows through the atomizer, the atomizing liquid flows from the liquid storage to the atomizing element, being heated, gasified and mixes with the air that flows into the atomizer to form an aerosol, then flows out of the atomizer from the suction nozzle.

To ensure the smooth flow of atomizing liquid out of the liquid storage in the process above, it is necessary to balance the air pressure inside and outside the liquid storage, that is, air needs to be constantly supplied into the liquid storage. Therefore, air exchange structure is usually set in the atomizer. A typical air exchange structure, namely, a kind of atomizing element bracket disclosed in patent No. CN216961537U, is shown in Fig. 1, wherein the tortuous groove marked with a is the air exchange structure. A similar devices is disclosed in CN115104780 A.

The design disclosed above is only suitable for atomizing liquid of low viscosity, but not for atomizing liquid of high viscosity (more than 10 Pa·s (10,000 cps) at the room temperature of 25°C). When processing atomizing liquid of low viscosity, it is necessary to increase the resistance to leakage as much as possible in order to prevent atomizing liquid from leaking into the air path. So air exchange structure a is designed as narrow, long and tortuous outside the atomizing element bracket. Because there is no need to worry about the fluidity of condensate and atomizing liquid while processing atomizing liquid of low viscosity, most of the atomizing liquid in the air exchanging pathway would flow back to the liquid storage under the action of pressure even if a small amount of atomizing liquid enters the air exchanging pathway when the pressure and amount of atomizing liquid in the storage decrease.

However, in the scenario of highly viscous atomizing liquid, the resistance to air bubble rise is already large, and the narrowness and length of the air path would increase the resistance. Moreover, once the atomizing liquid appears in the inner part again, poor liquidity of the viscous atomizing liquid will block the air path and further increase the resistance to the air bubble, then disable the bubbles to enter the liquid storage via passing through the atomizing liquid. This would cause block to the fluid, which will cause the dry ashing and burnout of atomizing element, harmful substances under high temperature will be released.

### Summary of This Invention

The invention is defined by the appended claims.

In view of the shortcomings of the prior art, the present disclosure aims to provide an atomizer characterized in that it comprises a suction nozzle, a shell, and a bottom cover; the shell includes a liquid storage, the atomizing element bracket is arranged below the liquid storage, and the atomizing element bracket comprises a liquid inlet; an atomizing element installation bin is arranged below the liquid inlet, and the atomizing element assembly is installed there; at least one air exchanging pathway is arranged between the inner wall of the atomizing element installation bin and the atomizing element assembly; the upper end of the air exchanging pathway is connected with the liquid storage, and its lower end is connected with the air inlet of the atomizer.

Preferably, a groove is arranged on the inner wall of the atomizing element installation bin; the groove and the outer wall of the atomizing element assembly form the air exchanging pathway in the atomizer.

Preferably, a groove is arranged on the outer wall of the atomizing element assembly; the groove and the inner wall of the atomizing element installation bin form the air exchanging pathway in the atomizer.

This disclosure also provides an atomizer characterized in that it comprises a suction nozzle, a shell, and a bottom cover; the shell includes a liquid storage, the atomizing element bracket is arranged below the liquid storage, and the atomizing element bracket comprises a liquid inlet; an atomizing element installation bin is arranged below the liquid inlet, and the atomizing element assembly is installed in the atomizing element installation bin; the atomizing element assembly comprises an atomizing element and an atomizing element seal; at least one air exchanging pathway is arranged between the atomizing element seal and the atomizing element; the upper end of the air exchanging pathway is connected with the liquid storage, and its lower end is connected with the air inlet of the atomizer.

Preferably, a groove is arranged on the wall of the atomizing element seal facing the atomizing element, thus the air exchanging pathway is formed between the groove and the outer wall of the atomizing element in the atomizer.

Preferably, the air exchanging pathway ranges from 0.015~0.04 square millimeter by caliber in the atomizer.

Preferably, the part of the air exchanging pathway near the liquid storage is perpendicular to the down-flowing direction of the atomized liquid in the atomizer.

Preferably, the atomizing element assembly is a cuboid in the atomizer.

The atomizer of the invention improves the design of air exchanging pathway, and solves the problems of the existing atomizer, including poor air exchange and difficulty in discharging liquid in the application scenario of highly viscous atomizing liquid.

### Brief Description of the Figures

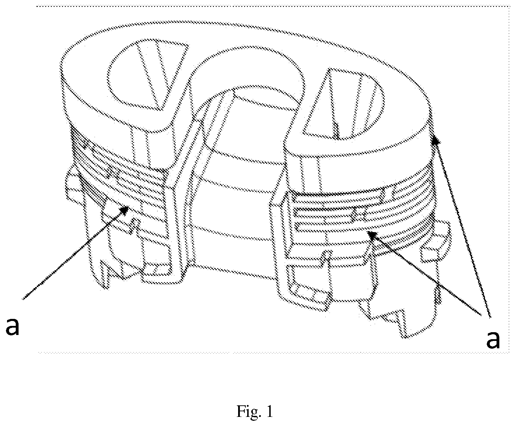

Fig. 1 is the schematic diagram on the air exchange structure of the atomizer by the prior art;
Fig. 2 is the stereogram of an atomizer invented this time;
Fig. 3 is the front view of the atomizer in Fig. 2;
Fig. 4 is a sectional view of the first embodiment along Line C-C in Fig. 3;
Fig. 5 is an exploded view of the atomizing element bracket and atomizing element assembly in Fig. 4;

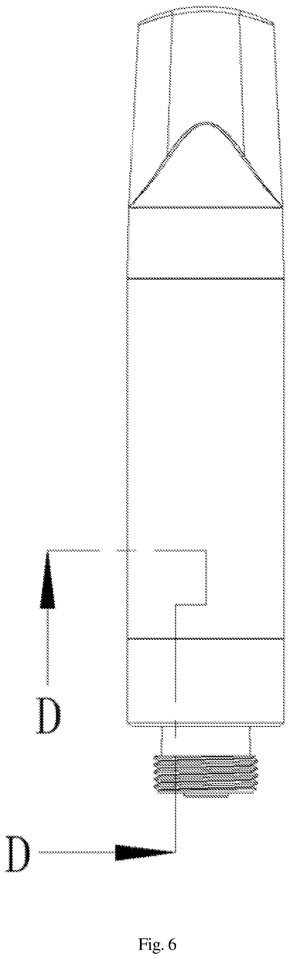

Fig. 6 is the left view of the atomizer in Fig. 2;

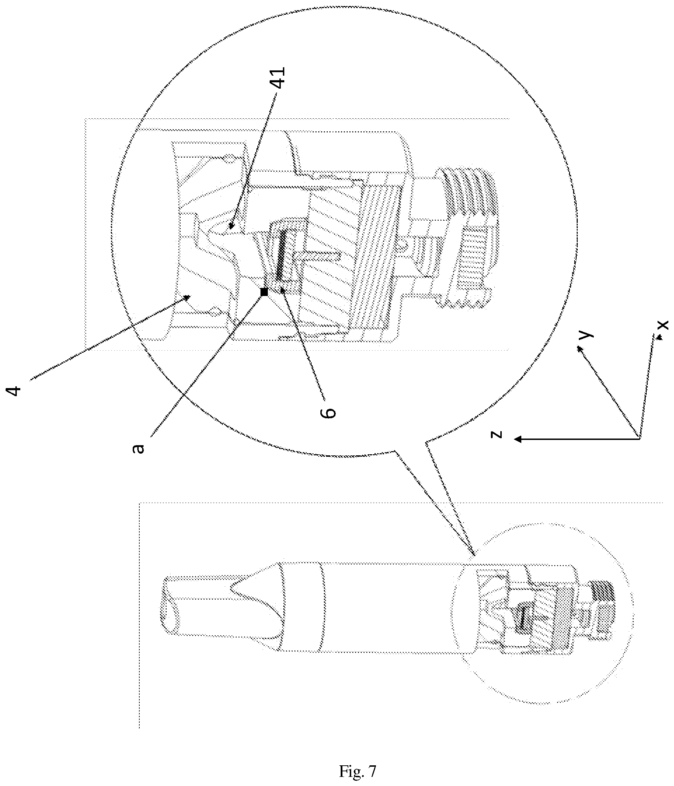

Fig. 7 is an exploded view of the second embodiment along Line D-D in Fig. 6;
Fig. 8 is an exploded view of the atomizing element bracket and atomizing element assembly in Fig. 7;
Fig. 9 is an exploded view of the atomizing element bracket and atomizing element assembly after the third in embodiment split along Line C-C in Fig. 3;
Fig. 10 is the stereogram of the atomizing element bracket and atomizing element assembly in the fourth embodiment;
Fig. 11 is a sectional view of the atomizing element bracket and atomizing element assembly in the fifth embodiment;
Fig. 12 is a sectional view of the atomizing element assembly in the sixth embodiment.

### Embodiments

The execution of this technology is explained by specific embodiments below, and a person skilled in the art can easily understand other advantages and effects from the summary disclosed herein. The technology can also be implemented or applied in other different ways, and the summary herein can also be modified or changed in various ways based on different views and applications .

Please refer to the attached figures then. To be sure, the figures provided in this embodiment are only explaining the basic conception of this technology, and thus do not show the actual number, size and shape of the component put into practice but only showing the component associated with the technology; the type, number and proportion of the components actually put into practice can be a random change, and the component layout pattern may also be more complex. Unless otherwise specified, the words "up", "down", "left", and "right" used herein are all from the perspective of the observer in the attached figure.

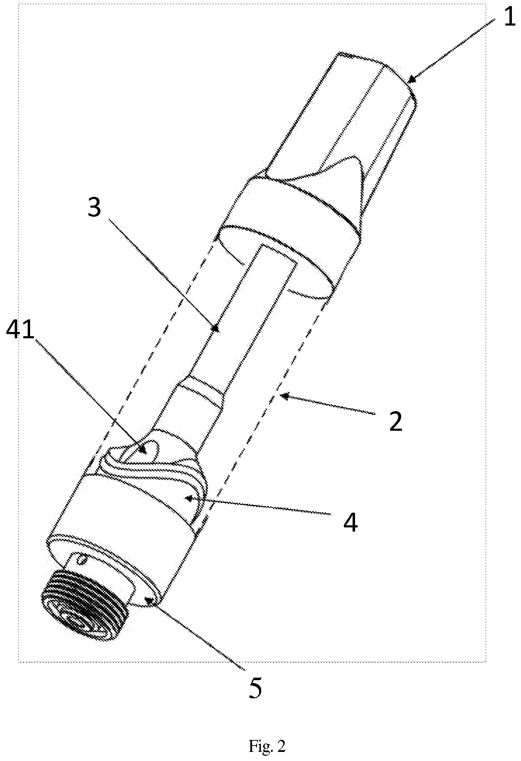

Firstly, please refer to Fig. 2, the structure diagram of the atomizer, which comprises suction nozzle 1, shell 2, outlet passage 3, atomizing element bracket 4, and bottom cover 5. To clearly display the internal structure of the atomizer, shell 2 is represented by a dotted line. The lower end of outlet passage 3 is connected with the air outlet at the top of the atomizing element bracket 4. The outer wall of outlet passage 3, the shell 2, and the upper surface of atomizing element bracket 4 together form a liquid storage for storing the atomized liquid. And the atomizing element bracket 4 comprises liquid inlet 41.

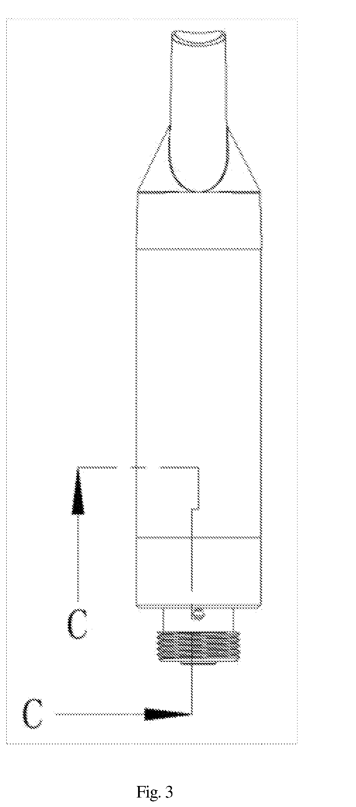

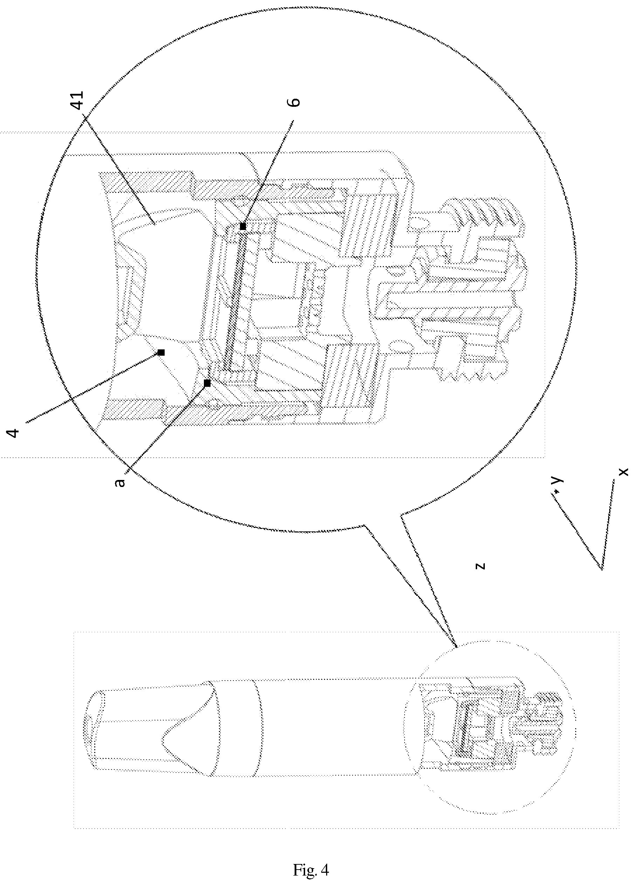

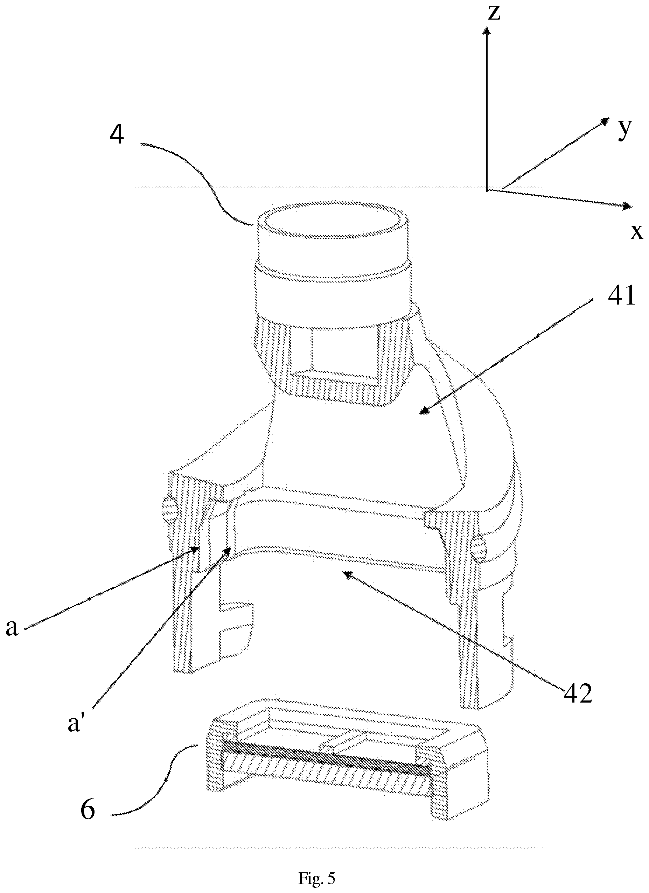

Then please refer to figures 3~5. Fig. 4 is the sectional view along Line C-C in Fig. 3, and Fig. 5 is an exploded view of atomizing element bracket 4 and atomizing element assembly 6 in Fig. 4. As shown in the figure, atomizing element installation bin 42 is arranged below atomizing element bracket 4 to install atomizing element assembly 6, which comprises the atomizing element for heating.

In this embodiment, the air exchange structure is also represented by mark a, and arranged between the inner wall of atomizing element bracket 4 and atomizing element assembly 6. The upper end of air exchanging pathway a is connected to liquid inlet 41, thus to the liquid storage, and its lower end is connected to the air inlet of the atomizer. Air exchanging pathway a is arranged between the inner wall of atomizing element installation bin 42 and atomizing element assembly 6. Compared with the prior design in which the air exchanging pathway is arranged outside the atomizing element bracket, the distance between air exchanging pathway a and the heating component is greatly reduced, thus the atomized liquid at the outlet of air exchanging pathway a would be less viscous in the preheating and heating process of the heating component, so that the bubbles in the groove are easy to escape upward.

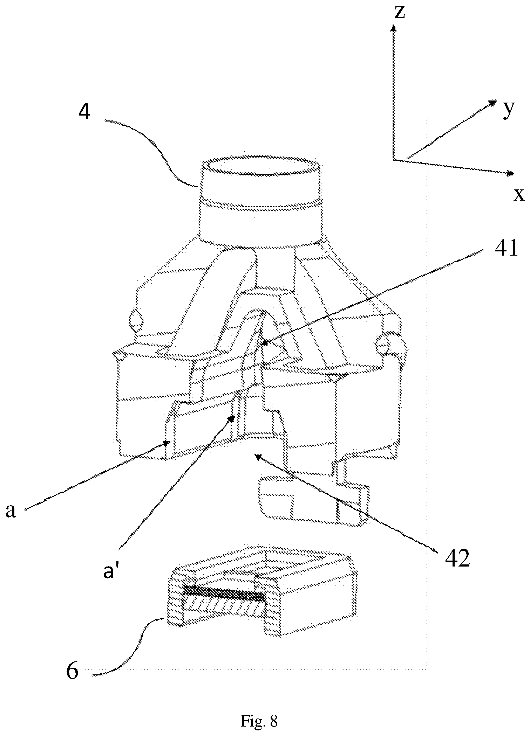

The first embodiment of air exchanging pathway a is shown in Fig. 5. In this embodiment, the atomizing element installation bin is a cuboid, and a groove is set on the short side of the inner wall of atomizing installation bin 42, while the outer surface of atomizing element assembly 6 is flat; in the second embodiment shown in Fig. 8, a groove is provided on the long side of the inner wall of atomizing element installation bin 42. In this way, air exchanging pathway a will be formed between atomizing element assembly 6 and the inner wall of atomizing element installation bin 42. It is understandable that multiple air exchanging pathways can be set up on the inner wall of the installation bin 42, extra air exchanging pathway(s) is(are) represented by mark a' in Fig. 5 and Fig. 8.

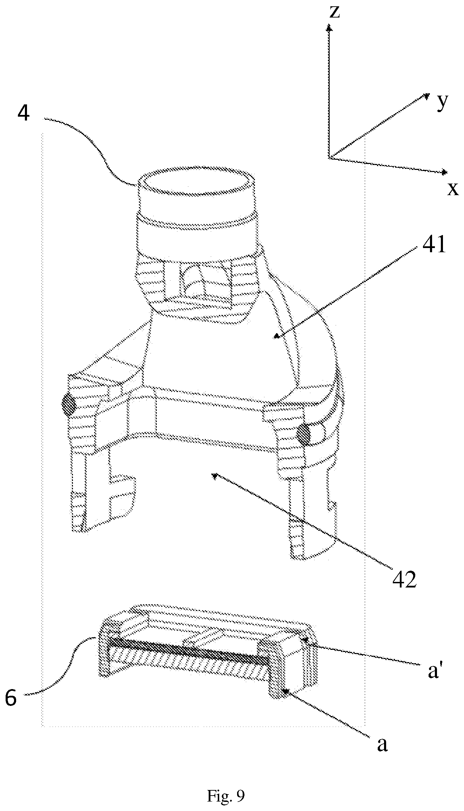

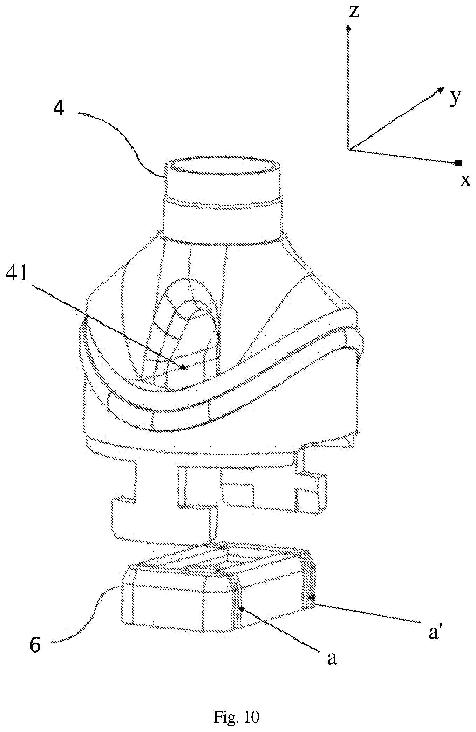

The third and fourth embodiments of air exchanging pathway a are shown in Fig. 9 and Fig. 10, that is, a groove is set on the outer surface of atomizing element assembly 6, and the inner wall of atomizing element installation bin 42 is flat; in the third embodiment, the groove is set on the short side of the outer wall of atomizing element assembly 6; in the fourth embodiment, the groove is set on the long side of the outer wall of atomizing element assembly 6. Then, air exchanging pathway a (and a') would be formed between atomizing element assembly 6 and the inner wall of atomizing element installation bin 42.

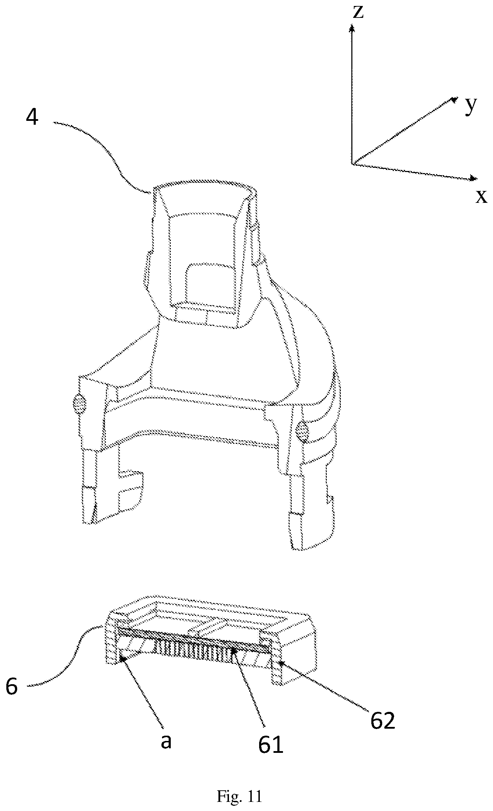

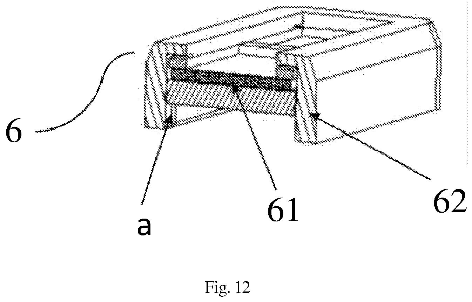

The fifth and sixth embodiments of air exchanging pathway a are shown in Fig. 11 and Fig. 12, wherein, atomizing element assembly 6 comprises atomizing element 61 and seal 62. Groove a is set on the side of seal 62 facing atomizing element 61. In the fifth embodiment, a groove is arranged on the side of seal 62 facing the short side of atomizing element 61; in the sixth embodiment, a groove is arranged on the side of seal 62 facing the long side of atomizing element 61.

As a preferred embodiment, the shape of air exchanging pathway a can also be determined by the intersection line between the vertical plane (x-z in Fig. 4 and Fig. 5) and the inner wall of atomizing element installation bin 42, as shown in Fig. 5 and Fig. 8. In this way, air exchanging pathway a has the shortest length and vertical directivity, the route of bubble filling is shortened, bubble blockage is less possible, the formation of condensate is reduced, and automatic unblocking is achievable even if there is condensate since it can sink away from the air exchanging pathway under the action of gravity. According to the preferred embodiment, multiple air exchanging pathways can be set to further ensure that even if some air exchanging pathways are blocked, the liquid storage can still be successfully filled with air.

Moreover, atomizing element installation bin 42 is a cuboid in the above embodiments, but this does not constitute a limitation. Atomizing element installation bin 42 can be set into other common shapes such as semi-sphere or oval based on actual needs, but the setting mode of air exchanging pathway a is still applicable.

The air exchanging pathway is connected to the liquid storage. In order to avoid liquid leakage, the air exchanging pathway shall be kept away from the liquid down-flowing direction near connection point on the one hand. For example, the part of the air exchanging pathway that near the liquid storage bin is set perpendicular to the direction of liquid down flowing in this embodiment; the caliber of the air exchanging pathway (expressed by its section area) shall be controlled. Generally speaking, the caliber of the air exchanging pathway can be appropriately expanded for the atomizing liquid with higher viscosity. The section of the air exchanging pathway in the present invention is a rectangle, with the long side ranging from 0.3-0.5 mm, and the width from 0.05-0.08 mm. Therefore, the caliber ranges from 0.015~0.04 square millimeter on the other hand. For air exchanging pathways of other section shapes, the above caliber range can be referred to.

The above embodiments are illustrative only of the principle and effectiveness of the technology and are not intended to limit the invention.

## Claims

1. An atomizer comprising
a suction nozzle (1), a shell (2), and a bottom cover (5);
the shell including a liquid storage, and an atomizing element bracket (4) arranged below the liquid storage, wherein the atomizing element bracket comprises a liquid inlet (41) that is fluidly connected to the liquid storage;
wherein the atomizer further comprises an atomizing element installation bin (42) arranged in the atomizing element bracket below the liquid inlet, and an atomizing element assembly (6) sealingly installed therein, the atomizing element assembly comprising an atomizing element (61) and an atomizing element seal (62); wherein at least one air exchanging pathway (a) is arranged to bypass the atomizing element seal, the at least one air exchanging pathway being arranged either between an inner wall of the atomizing element installation bin and the atomizing element seal or between the atomizing element seal and the atomizing element; wherein the upper end of the at least one air exchanging pathway is connected with the liquid storage, and its lower end is disposed inside the atomizing element bracket below the atomizing element assembly and connected with an air inlet of the atomizer.

2. The atomizer according to claim 1, **characterized in that** a groove is arranged on an inner wall of the atomizing element installation bin; wherein the groove and an outer wall of the atomizing element seal form the at least one air exchanging pathway.

3. The atomizer according to claim 1, **characterized in that** a groove is arranged on an outer wall of the atomizing element seal; wherein the groove and an inner wall of the atomizing element installation bin form the at least one air exchanging pathway.

4. The atomizer according to claim 1,**characterized in that** a groove is arranged on a wall of the atomizing element seal facing the atomizing element, wherein the at least one air exchanging pathway is formed between the groove and the outer wall of the atomizing element.

5. The atomizer according to any one of claims 1 to 4, **characterized in that** the at least one air exchanging pathway ranges from 0.015~0.04 square millimeter by caliber.

6. The atomizer according to any one of claims 1 to 5, **characterized in that** a part of the at least one air exchanging pathway near the liquid storage is perpendicular to a down-flowing direction of the atomizing liquid.

7. The atomizer according to any one of claims 1 to 6, **characterized in that** the atomizing element assembly is a cuboid.

8. The atomizer according to claim 7, **characterized in that** there are four air exchanging pathways.

9. The atomizer according to claim 8, **characterized in that** the long side or short side of the atomizing element installation bin is provided with two air exchanging pathways.

10. The atomizer according to any one of claims 1 to 9, **characterized in that** the atomizer is adapted for atomizing a high viscosity liquid of greater than 10 Pa·s (10,000 cP) at a temperature of 25 degrees Celsius.

## Patentansprüche

1. Zerstäuber, der Folgendes umfasst:
eine Saugdüse (1), eine Hülle (2) und eine untere Abdeckung (5);
wobei die Hülle einen Flüssigkeitsspeicher und eine unter dem Flüssigkeitsspeicher angeordnete Zerstäubungselementhalterung (4) enthält, wobei die Zerstäubungselementhalterung einen Flüssigkeitseinlass (41) umfasst, der mit dem Flüssigkeitsspeicher fluidverbunden ist;
wobei der Zerstäuber ferner Folgendes umfasst:
einen Zerstäubungselement-Installationsbehälter (42), der in der Zerstäubungselementhalterung unterhalb des Flüssigkeitseinlasses angeordnet ist, und eine darin dichtend installierte Zerstäubungselementanordnung (6), wobei die Zerstäubungselementanordnung ein Zerstäubungselement (61) und eine Zerstäubungselementdichtung (62) umfasst; wobei mindestens ein Luftaustauschweg (a) so angeordnet ist, dass er die Zerstäubungselementdichtung umgeht, wobei der mindestens eine Luftaustauschweg entweder zwischen einer Innenwand des Zerstäubungselement-Installationsbehälters und der Zerstäubungselementdichtung oder zwischen der Zerstäubungselementdichtung und dem Zerstäubungselement angeordnet ist; wobei das obere Ende des mindestens einen Luftaustauschwegs mit dem Flüssigkeitsspeicher verbunden ist und sein unteres Ende innerhalb der Zerstäubungselementhalterung unterhalb der Zerstäubungselementanordnung angeordnet und mit einem Lufteinlass des Zerstäubers verbunden ist.

2. Zerstäuber nach Anspruch 1, **dadurch gekennzeichnet, dass** an einer Innenwand des Zerstäubungselement-Installationsbehälters eine Nut angeordnet ist; wobei die Nut und eine Außenwand der Zerstäubungselementdichtung den mindestens einen Luftaustauschweg bilden.

3. Zerstäuber nach Anspruch 1, **dadurch gekennzeichnet, dass** an einer Außenwand der Zerstäubungselementdichtung eine Nut angeordnet ist; wobei die Nut und eine Innenwand des Zerstäubungselement-Installationsbehälters den mindestens einen Luftaustauschweg bilden.

4. Zerstäuber nach Anspruch 1, **dadurch gekennzeichnet, dass** an einer dem Zerstäubungselement zugewandten Wand der Zerstäubungselementdichtung eine Nut angeordnet ist, wobei der mindestens eine Luftaustauschweg zwischen der Nut und der Außenwand des Zerstäubungselements ausgebildet ist.

5. Zerstäuber nach einem der Ansprüche 1 bis 4, **dadurch gekennzeichnet, dass** der mindestens eine Luftaustauschweg einen Durchmesser von 0,015-0,04 Quadratmillimetern aufweist.

6. Zerstäuber nach einem der Ansprüche 1 bis 5, **dadurch gekennzeichnet, dass** ein Teil des mindestens einen Luftaustauschwegs in der Nähe des Flüssigkeitsspeichers senkrecht zur Abwärtsströmungsrichtung der Zerstäubungsflüssigkeit verläuft.

7. Zerstäuber nach einem der Ansprüche 1 bis 6, **dadurch gekennzeichnet, dass** die Zerstäubungselementanordnung ein Quader ist.

8. Zerstäuber nach Anspruch 7, **dadurch gekennzeichnet, dass** es vier Luftaustauschwege gibt.

9. Zerstäuber nach Anspruch 8, **dadurch gekennzeichnet, dass** die lange Seite oder kurze Seite des Zerstäubungselement-Installationsbehälters mit zwei Luftaustauschwegen versehen ist.

10. Zerstäuber nach einem der Ansprüche 1 bis 9, **dadurch gekennzeichnet, dass** der Zerstäuber zum Zerstäuben einer hochviskosen Flüssigkeit von mehr als 10 Pa·s (10.000 cP) bei einer Temperatur von 25 Grad Celsius geeignet ist.

## Revendications

1. Atomiseur comprenant
une buse d'aspiration (1), une coque (2), et un couvercle inférieur (5) ;
la coque incluant un réservoir de liquide, et un support d'élément atomiseur (4) agencé sous le réservoir de liquide, où le support d'élément atomiseur comprend un orifice d'entrée de liquide (41) qui est relié fluidiquement au réservoir de liquide ;
où l'atomiseur comprend en outre
un bac d'installation d'élément atomiseur (42) agencé dans le support d'élément atomiseur sous l'orifice d'entrée de liquide, et un ensemble d'élément atomiseur (6) installé de manière étanche à l'intérieur de celui-ci, l'ensemble d'élément atomiseur comprenant un élément atomiseur (61) et un joint d'étanchéité d'élément atomiseur (62) ; où au moins un conduit d'échange d'air (a) est agencé pour contourner le joint d'étanchéité d'élément atomiseur, l'au moins un conduit d'échange d'air étant agencé soit entre une paroi interne du bac d'installation d'élément atomiseur et le joint d'étanchéité d'élément atomiseur soit entre le joint d'étanchéité d'élément atomiseur et l'élément atomiseur ; où l'extrémité supérieure de l'au moins un conduit d'échange d'air est reliée au réservoir de liquide, et son extrémité inférieure est disposée à l'intérieur du support d'élément atomiseur sous l'ensemble d'élément atomiseur et reliée à un orifice d'entrée d'air de l'atomiseur.

2. Atomiseur selon la revendication 1, **caractérisé en ce qu'**une rainure est agencée sur une paroi interne du bac d'installation d'élément atomiseur ; où la rainure et une paroi externe du joint d'étanchéité d'élément atomiseur forment l'au moins un conduit d'échange d'air.

3. Atomiseur selon la revendication 1, **caractérisé en ce qu'**une rainure est agencée sur une paroi externe du joint d'étanchéité d'élément atomiseur ; où la rainure et une paroi interne du bac d'installation d'élément atomiseur forment l'au moins un conduit d'échange d'air.

4. Atomiseur selon la revendication 1, **caractérisé en ce qu'**une rainure est agencée sur une paroi du joint d'étanchéité d'élément atomiseur faisant face à l'élément atomiseur, où l'au moins un conduit d'échange d'air est formé entre la rainure et la paroi externe de l'élément atomiseur.

5. Atomiseur selon l'une quelconque des revendications 1 à 4, **caractérisé en ce que** l'au moins un conduit d'échange d'air varie de 0,015 à 0,04 millimètre carré par calibre.

6. Atomiseur selon l'une quelconque des revendications 1 à 5, **caractérisé en ce qu'**une partie de l'au moins un conduit d'échange d'air près du réservoir de liquide est perpendiculaire à une direction d'écoulement descendante du liquide d'atomisation.

7. Atomiseur selon l'une quelconque des revendications 1 à 6, **caractérisé en ce que** l'ensemble d'élément atomiseur est un cuboïde.

8. Atomiseur selon la revendication 7, **caractérisé en ce qu'**il existe quatre conduits d'échange d'air.

9. Atomiseur selon la revendication 8, **caractérisé en ce que** le côté long ou le côté court du bac d'installation d'élément atomiseur est muni de deux conduits d'échange d'air.

10. Atomiseur selon l'une quelconque des revendications 1 à 9, **caractérisé en ce que** l'atomiseur est adapté pour atomiser un liquide à haute viscosité supérieure à 10 Pa·s (10 000 cP) à une température de 25 degrés Celsius.
